# EUROPÄISCHE PATENTSCHRIFT

(11) **EP 0 412 447 B1**
(45) Veröffentlichungstag und Bekanntmachung des Hinweises auf die Patenterteilung: **15.06.1994**
(21) Anmeldenummer: 90114934.4
(22) Anmeldetag: 03.08.1990
(51) Int. Cl.: D01B 3/02, G01N 33/36, G01V 9/04, B07C 5/34

(54) **Verfahren und Vorrichtung zur Ermittlung von mit Fremdkörpern verunreinigtem Fasergut**
Method and apparatus for controlling fibre material contaminated with foreign matter
Procédé et appareil pour détecter des corps étrangers dans la matière de fibres textiles

(30) Priorität: 09.08.1989 DE 3926346
(43) Veröffentlichungstag der Anmeldung: 13.02.1991
(73) Patentinhaber: MASCHINENFABRIK RIETER AG, CH-8406 Winterthur (CH)
(72) Erfinder: Demuth, Robert, CH-8309 Nuerensdorf (CH); Faas, Jürg, CH-8474 Dinhard (CH)

(56) Entgegenhaltungen:
- EP-A- 0 285 602
- DE-A- 3 708 188
- FR-A- 2 609 058
- FR-A- 2 610 725
- GB-A- 2 210 907

## Beschreibung

Die vorliegende Erfindung betrifft ein Verfahren bzw. eine Vorrichtung zur Ermittlung von mit Fremdkörpern verunreinigtem Fasergut.

Unter Fremdkörpern in Baumwolle versteht man
a) Schmutz,
b) Mineralien, beispielsweise Steine,
c) Metallteile,
d) Fremdfasern und Folien,
welche beispielsweise Verpackungsreste sowie Jutesackreste, Plastikfolienreste, Schnüre oder Bänder, Lumpen oder Putzfäden umfassen.

Schwierigkeiten in Spinnereien verursachen heute vor allem Fremdfasern und Fremdfolien, da es für diese Kategorie noch keine erprobte Ausscheidungseinrichtung gibt. Die anderen Arten von Fremdkörpern, d.h. Schmutz, Mineralien und Metallteile können nach dem heutigen Stand der Technik zufriedenstellend ausgeschieden werden, selbst wenn dies in manchen Fällen mit einem gleichzeitigen unerwünschten Verlust an Fasergut verbunden ist. Sind aber Fremdfaser oder Folienreste vorhanden, so führen diese zu negativen Auswirkungen in der Spinnerei. Beispielsweise tritt ein schlechtes Laufverhalten der Spinnmaschinen und erhöhte Fadenbruchrate auf und es entstehen Garnfehler in Form von Schwachstellen, Dünn-/Dickstellen und Färbungsfehler. Auch können Fremdfasern zu einer Beschädigung von Garnituren in Reinigungsmaschinen führen, obwohl dies eher selten vorkommt.

Es sind schon verschiedene Vorschläge gemacht worden, wie man Fremdfasern ausscheidet. Die meisten Vorschläge zielen darauf, die Faserflocken und Fremdfaserstoffe in einen Faserstrom aufzulösen, die Fremdfasern zu orten und dann mit einem Teil des Faserstroms auszuscheiden. Als Beispiel kann hier auf die DE-A-3644535 hingewiesen werden. Ein jüngerer nicht vorveröffentlichter Vorschlag dieser Art ist aus EP-A-0364786 der Maschinenfabrik Rieter AG zu entnehmen. Auch die EP-A-285602 beschreibt die Erkennung und Ausscheidung von Fremdkörpern aus einem aufgelockerten Fasermaterial mit einem Flockengewicht von 10⁻¹ bis 10⁻³ g/Flocke, das nach der Ballenabtragung erreicht wird.

Der vorliegenden Erfindung liegt die Aufgabe zugrunde, das Problem der Fremdfasern aus einer ganz anderen Richtung zu betrachten und anstatt aufwendige Versuche anzustellen, die Fremdfasern vollautomatisch auszuscheiden, ein Verfahren bzw. eine Vorrichtung zu schaffen, das bzw. die eine einwandfreie Ermittlung der Verunreinigung der Faserballen mit Fremdfasern ermöglicht, so daß man bei Feststellung eines unakzeptablen Grades der Verunreinigung am Faserballen mit Fremdfasern vom Lieferant einen Preisnachlaß erreichen kann.

Mit anderen Worten will die Erfindung eine Ursachenbekämpfung dadurch betreiben, daß sie die Voraussetzungen schafft, um stark fremdfaserbehaftete Ballen zu erkennen, mit dem Ziel, den Baumwollieferanten und den Händler zu belangen und eine Rückgabe der gelieferten Ballen oder eine Lieferpreisreduktion zu erreichen, wodurch die entsprechenden Forderungen gegen den Händler bzw. den Baumwollieferanten zu Maßnahmen führen sollen, die das Entstehen der Verschmutzung an der Quelle der Verschmutzung bekämpft.

Mit anderen Worten wird davon ausgegangen, daß aufgrund von Fremdfasern keine "fatalen" Folgefehler in der Spinnerei, wie Beschädigungen an den Anlagen auftreten, sondern die Fremdfasern führen zu einer geringen Qualität des Garnes, was durch einen Preisnachlaß für das Rohmaterial ausgeglichen werden kann.

Weiterhin schafft die vorliegende Erfindung die Voraussetzung dafür, daß auch Baumwollieferanten selbst das gelieferte Baumwollmaterial wenigstens stichprobenartig nach Verunreinigungen durch Fremdfasern und Fremdfolien absuchen können.

Ausgehend von der DE-A-3 436 498 wird zur Lösung der erfindungsgemäßen Aufgabe verfahrensmäßig vorgeschlagen, daß
- weiche Fremdkörper in Form von Fremdfasern und Folienresten, insbesondere Verpackungsresten, so wie Jutesackreste, Plastikfolienreste, Schnüre oder Bänder, Lumpen oder Putzfäden ermittelt werden und das Fasergut daraufhin an der Oberfläche der Ballen untersucht wird,
- die Untersuchung durch eine bildliche Aufnahme der Oberfläche wenigstens einiger der durch die Ballenabtragung freigelegten Fasergutschichten über die gesamte Breite der Faserballen erfolgt, und
- das Ausmass der Verunreinigung mit den abgetasteten Fremdkörpern der genannten Form festgehalten und in Zuordnung zu den jeweiligen Ballen gespeichert wird.

Ausgehend von der DE-A-3 436 498 sieht die Erfindung Verrichtungsmäßig vor, dass ein Abtastgerät vorgesehen ist, welches die Fremdkörper in Form von Fremdfasern und Folienresten, insbesondere Verpackungsresten, so wie Jutesackreste, Plastikfolienreste, Schnüre oder Bänder, Lumpen oder Putzfäden an der bei der Ballenabtragung schichtweise freigelegten Oberfläche der einzelnen Faserballen über die gesamte Breite der Faserballen detektiert und in Bildern festhält, sowie eine Speichereinrichtung zur Speicherung der durch die Abtastung gewonnenen Bilder, die jeweils einem Ballen zugeordnet sind.

Mit diesen Vorschlägen wird erkannt, daß die typischen Fremdfaserverunreinigungen an der Ballenoberfläche bei der Ballenabtragung zum Vorschein kommen, wobei der schichtweise Abbau der einzelnen Ballen eine stichprobenartige Untersuchung der einzelnen Ballen auf deren Fremdfaserinhalt ermöglicht. Weiterhin ist bei der Ballenabtragung eine eindeutige Zuordnung der festgestellten Verunreinigungen zu den jeweiligen Ballen möglich, so daß man ein einwandfreies Beweismittel für spätere Reklamationen schaffen kann.

Es ist zwar bereits aus der DE-A 34 36 498 bekannt, Fremdkörper in Form von Metallteilen wie Bandeisen- und Drahtstücken, Werkzeugen und dergleichen in den Textilfaserballen mittels Fremdkörpersuchgeräten zu ermitteln, dieser Vorschlag zielt jedoch darauf, die Fremdkörper, die im Inneren des Faserballens vorhanden sind, mittels die Faserballen durchdringenden Strahlungen wie Röntgenstrahlen fest zustellen und dann zu entfernen. Die Schrift befaßt sich jedoch nicht mit der Feststellung von Fremdfasern und nützt auch nicht die Gelegenheit aus, wie bei der vorliegenden Erfindung, die an der freigelegten Oberfläche der Ballen feststellbaren Fremdfasern zu erfassen.

Die vorliegende Erfindung erkennt weiterhin an, daß das Ausmaß der Verunreinigung durch die Anzahl der abgetasteten Fremdkörper und/oder die Ausdehnung der Fremdkörper und/oder die Farbe der Fremdkörper und/oder durch einen diesen Parametern entsprechenden Wert angegeben werden kann.

Bei einer Ballenabtragmaschine mit einem Turm, der entlang einer Ballenreihe fahrbar ist und ein in einem Arm der Ballenabtragmaschine untergebrachtes Abtragorgan trägt, kann das Abtasten der freigelegten Oberflächen mittels einer am Turm oder Arm der Ballenabtragmaschine und vorzugsweise in Abtragrichtung vor dem im Arm untergebrachten Abtragorgan wirkungsvoll durchgeführt werden. Die Anbringung der Abtasteinrichtung in Abtragrichtung vor dem Abtragorgan hat den Vorteil, daß hier eine besonders ruhige Oberfläche vorliegt, so daß eine Störung des Abtastens durch die Arbeit des Abtragorgans vermieden werden kann. Bei einer Ballenabtragmaschine, die feststeht und Material von in Richtung der Ballenabtragmaschine vorgeschobenen Ballen abträgt, kann die Abtasteinrichtung an feststehender Struktur angebracht werden, obwohl sie auch hier evtl. am Gehäuse eines hin- und herfahrbaren Abtragorganes angebracht werden kann.

Die Abtasteinrichtung arbeitet vorzugsweise auf elektrooptischer Basis. Beispielsweise kann das Verfahren so ausgelegt sein, daß die Oberfläche der freigelegten Fasergutschichten mit einer Videokamera abgebildet wird, und die so gewonnenen Videobilder als Nachweis für die Verunreinigung des Ballens gespeichert werden. Diese Speicherung kann im einfachsten Fall in Form eines Videofilms stattfinden, der auch eine Identifikation des abgetragenen Ballens, beispielsweise in Form einer Strichkodierung trägt. Es ist nämlich bereits bekannt, Faserballen mit Strichkodierungen zu versehen, die Angaben zu der Herkunft des Ballens und den Fasereigenschaften enthalten. Diese Strichkodierung könnte zum Zwecke der sicheren Erkennung des Ballens beim ersten Durchgang auf der Oberfläche des Ballens oder an der Ballenabtragmaschine im unmittelbaren Bereich des Ballens angebracht und von der Videokamera mit der Ballenoberfläche aufgenommen werden. Auch kann der Videofilm ohne weiteres kommentiert werden, damit auch auf diese Weise die Angaben zu der Herkunft der jeweiligen Ballen einwandfrei etabliert sind.

Es ist gar nicht notwendig, alle Videofilme anzusehen, um mit dem menschlichen Auge das Vorhandensein von Verunreinigungen in den Ballen zu erkennen. Stattdessen können die Videofilme vorübergehend gelagert werden, bis das entsprechende Fasergut zu Garn oder Webstoff verarbeitet worden ist. Nur im Falle der Feststellung von erhöhten Fehlern bei der Garnherstellung entsteht ein Grund, die gelagerten Videofilme anzuschauen. Auch hier leistet die vorliegende Erfindung Abhilfe, da man erfindungsgemäß vorzugsweise so vorgeht, daß das nachfolgende Herstellungsverfahren, bei dem das Fasergut zu Garn verarbeitet wird, so durchgeführt wird, daß eine Zuordnung zwischen dem Fasermaterial in den verschiedenen Verarbeitungsstufen und den jeweiligen Faserballen gegeben ist, wodurch bei der Feststellung von erhöhten Fehlern bei der Garnherstellung die den betreffenden Ballen zugeordneten Videoaufnahmen von einer Betriebsperson gezielt durchgeprüft werden können, um bei mäßigem Aufwand den Nachweis der Verunreinigung sicherzustellen.

Anstatt nur Videofilme zu speichern, besteht auch die Möglichkeit, die Oberfläche der freigelegten Fasergutschichten mit einer Videokamera abzubilden und die so gewonnenen Videosignale zu Fremdkörperverunreinigungssignalen zu verarbeiten und auszuwerten.

Bei der Videoaufnahme kann man, wie im Anspruch 8 vorgesehen, bei jedem Durchgang der Ballenabtragmaschine, d.h. bei jedem Abtragen der Fasergutschicht Videoaufnahmen von der gesamten Oberfläche jeder Schicht anfertigen. Es ist aber auch möglich, entsprechend dem Anspruch 9 Videoaufnahmen nur während einiger der Durchgänge der Ballenabtragmaschine anzufertigen, beispielsweise jedesmal nach dem Abtragen von Schichten einer bestimmten Dicke, beispielsweise jedesmal, wenn die einzelnen Ballen um 10 cm kürzer geworden sind. Dies umfaßt auch die Erkenntnis, daß es nicht notwendig ist, hundertprozentig die Verunreinigungen bestimmter Ballen zu erkennen, sondern nur einen Bruchteil der vorhandenen Fremdfaserverunreinigung, da man auch auf dieser Basis eine Statistik für den Verunreinigungsgrad und daher auch eine Vergleichsbasis für einzelne Ballen schaffen kann.

Eine besonders bevorzugte Verfahrensvariante zeichnet sich dadurch aus, daß wenigstens der von der Videokamera abgebildete Streifen der Oberfläche bzw. der entsprechende Flächenbereich mit diffusem Licht zumindest im wesentlichen gleichmäßig ausgeleuchtet wird. Diese Verfahrensvariante sorgt dafür, das auch ein guter Kontrast bei den Videoaufnahmen erzielt werden kann. Die Ausleuchtung erfolgt vorzugsweise mittels eines in der Brennlinie eines sich über die Breite der Ballen erstreckenden Hohlspiegels, beispielsweise eines Zylinderspiegels oder Parabolspiegels, angeordneten Leuchtrohres, wobei das Leuchtrohr bzw. die Brennlinie des länglichen Hohlspiegels quer zum Ballen und in einer horizontalen Ebene liegt. Wenn das Leuchtrohr eine spektrale Strahlungsverteilung aufweist, die sich von wenigstens etwa 400 bis etwa 700 nm erstreckt und vorzugsweise noch weiter in den Infrarotbereich und evtl. auch noch in den UV-Bereich hineingeht, kann die Videokamera dann eine herkömmliche Videokamera mit Aufnahmeeinrichtungen für unterschiedliche Spektralbereiche umfassen, beispielsweise die Spektralbereiche blau, grün und rot, wobei die Videosignale der drei Aufnahmeeinrichtungen getrennt ausgewertet werden können. Für die Auswertung des Videosignals bzw. der Videosignale stehen eine ganze Vielfalt von Möglichkeiten zur Verfügung. Beispielsweise kann man annehmen, daß bei gleichmäßiger Ausleuchtung einer aus reinen Baumwollfasern bestehenden Oberfläche diese als gleichmäßig heller Hintergrund vorliegt, so daß alle Schwankungen der Amplitude des Videosignals (der Videosignale) außerhalb eines festgelegten Schwellenwertbereiches auf eine Verunreinigung hinweisen. Die Fläche der Verunreinigung kann auch durch die Anzahl der betroffenen "Pixel" im Videobild ermittelt werden, wodurch der Grad der Verunreinigung verifiziert werden kann.

Durch Untersuchung der Videosignale in unterschiedlichen Farbbereichen werden die unterschiedlichen Eigenschaften der verschiedenen möglichen Verunreinigung in den unterschiedlichen Spektralbereichen ausgenützt, um diese Verunreinigungen sichtbar zu machen.

Eine weitere Möglichkeit zur Erhöhung des Wirkungsgrades der Abtasteinrichtung besteht darin, Polarisationsfilter vorzusehen, die zur Änderung bzw. Festlegung des Polarisationszustandes des vom Leuchtrohr abgestrahlten und/oder von der Videokamera aufgenommenen Lichtes dienen.

Das Abtasten der Ballenoberfläche kann auch mit anderen Mitteln erfolgen als mit Videokameraaufnahmen. Beispielsweise kann, entsprechend dem Anspruch 15, das Abtasten der freigelegten Oberfläche der Fasergutschichten mittels einer von der Abtragmaschine getragenen Ultraschallabtasteinrichtung vorgenommen werden. Weiterhin besteht die Möglichkeit, entsprechend dem Anspruch 16, das erfindungsgemäße Verfahren so durchzuführen, daß die freigelegte Oberfläche des Ballens aufgewärmt wird, beispielsweise mit Infrarotstrahlung oder mit Mikrowellenenergie, um ein Wärmebild der Oberfläche anzufertigen, aus dem der Grad der Verunreinigung ermittelt werden kann, beispielsweise durch die Flächenanteile des Bildes mit gegenüber dem Fasergut erhöhter oder herabgesetzter Temperatur.

Eine besonders wichtige Ausführungsform ist dem Anspruch 17 zu entnehmen. Eine weitere Möglichkeit besteht darin, bei Feststellung größerer Fremdkörper diese auszuscheiden, ggf. nach Unterbrechung des Abtragverfahrens.

Weitere vorteilhafte Ausbildungen der erfindungsgemäßen Vorrichtung sind den weiteren Unteransprüchen 19 bis 23 zu entnehmen.

Die vorliegende Erfindung wird nachfolgend anhand von Ausführungsbeispielen unter Bezugnahme auf die Zeichnung näher erläutert, in welcher zeigt:
- Fig. 1: eine Seitenansicht einer Ballenabtragmaschine mit einem entlang einer Ballenreihe fahrbaren Turm und einer am Turm bzw. am Arm der Ballenabtragmaschine montierten Videokamera,
- Fig. 2: einen Seitenansicht einer Ballenabtragmaschine entsprechend der Fig. 1, jedoch mit einer Abtasteinrichtung in Form eines Ultraschall-Senders/-Empfängers, und
- Fig. 3: eine weitere Seitenansicht einer Ballenabtragmaschine entsprechend der Fig. 1, jedoch mit einer Wärmequelle und einer ein Wärmebild erzeugenden Abtasteinrichtung.

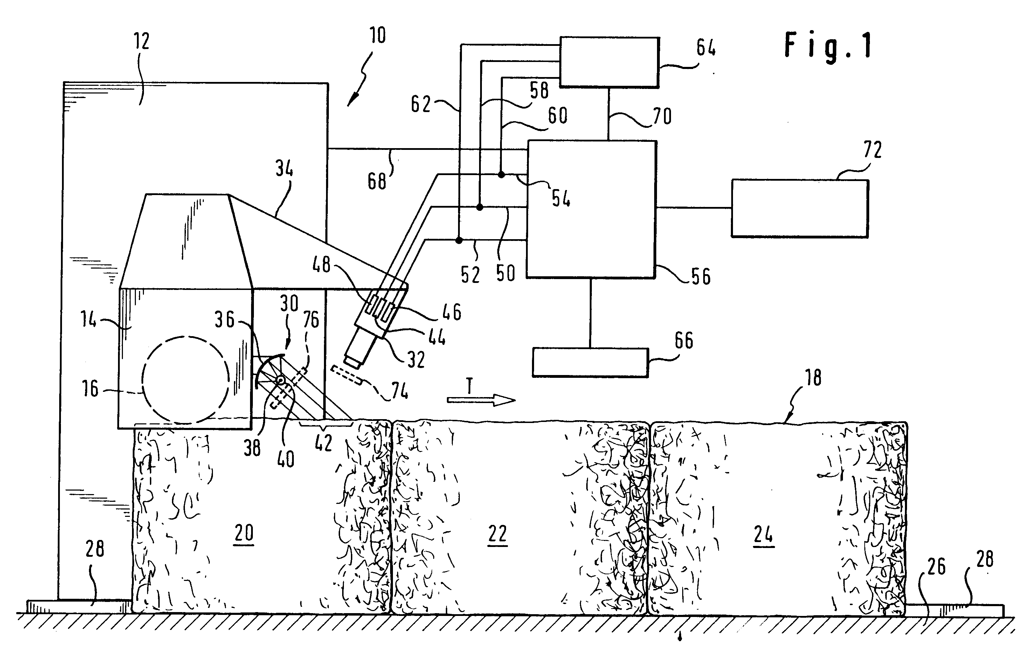

Fig. 1 zeigt eine Ballenabtragmaschine 10 mit einem Turm 12, der auf der einen Seite einen Arm 14 aufweist, in dem ein drehbares Abtragorgan 16, beispielsweise ein mit Zahnscheiben ausgestatteter Rotor untergebracht ist. Die Ballenabtragmaschine 10 dient zur Abtragung einer Ballenreihe 18, welche in diesem Beispiel aus drei Ballen 20, 22 und 24 besteht. Diese Ballen sind auf dem Boden nebeneinander aufgestellt und der Turm 12 kann über Räder (nicht gezeigt) entlang den am Boden 26 angebrachten Schienen 28 fahren, wobei das Abtragorgan 16 in an sich bekannter Weise die Ballenreihe 18 schichtweise abträgt. Das abgetragene, gleichzeitig vom Abtragorgan 16 aufgelöste Flockenmaterial wird in eine im oberen Teil des Abtragarmes 14 vorgesehenen Transportleitung gebracht, mittels der es einer nach der Ballenabtragmaschine vorgesehenen Reinigungsmaschine zugeführt wird.

Bei der in Fig. 1 gezeigten Ballenabtragmaschine wird nur in einer Richtung abgetragen, nämlich in Pfeilrichtung T. Vor dem Arm 14 montiert, befindet sich eine Beleuchtungseinrichtung 30 und eine Videokamera 32. Die Videokamera 32 ist mittels einer stabilen Struktur 34 vom Arm 14 getragen.

Wie aus Fig. 1 ersichtlich, besteht die Beleuchtungseinrichtung aus einem länglichen Hohlspiegel 36, der sich über die gesamte Breite der Ballenreihe erstreckt, d.h. in Fig. 1 senkrecht zu der Zeichnungsebene. Es handelt sich bei dem Hohlspiegel 36 um einen Zylinderspiegel mit einer Brennlinie 38, die sich ebenfalls quer über die Breite der Ballenreihe 18 erstreckt und in einer waagrechten Ebene liegt. Koaxial zu der Brennlinie angeordnet befindet sich ein Leuchtstoffrohr, beispielsweise ein Lumilux-Rohr der Firma Osram, Typ tw 19. Das Leuchtrohr 40 leuchtet sowohl direkt als auch indirekt über den Hohlspiegel 36 einen Bereich 42 der Ballenoberfläche gleichmäßig aus, wobei sich dieser Bereich ebenfalls über die gesamte Breite der Ballenreihe bzw. der einzelnen Ballen erstreckt. Die Videokamera 32 ist auf den ausgeleuchteten Bereich 42 gerichtet und fokussiert. Diese Kamera hat, wie üblich bei Farbvideokameras, drei Bildröhren, die jeweils Licht vom blauen, grünen und roten Teil des Spektrums aufnimmt. Durch die starke Beleuchtung wird die Oberfläche der Ballenreihe zum Selbstleuchter gemacht. Die drei Videofarbsignale von den drei Bildröhren 44, 46 und 48 werden über jeweilige Leitungen 50, 52 und 54 nach Umwandlung in Digitalsignale einem Computer 56 zugeführt. Weiterhin werden diese Videosignale über Leitungen 58, 60 und 62 an einem an sich bekannten Videoaufnahmegerät 64 angelegt, wobei das Gerät 64 eine Videokassette anfertigt, welche eine permanente Aufzeichnung der während der Ballenabtragung freigelegten Oberfläche der Ballenreihe darstellt.

Eine Tastatur 66 ermöglicht es der Betriebsperson, Angaben zu den einzelnen in der Reihe 18 aufgestellten Ballen 20 bis 24 in den Computer einzugeben, damit dieser eine einwandfreie Zuordnung der Videoaufnahmesignale an den Leitungen 50, 52 und 54 zu den einzelnen Ballen geben kann. Zu diesem Zweck erhält der Computer 56 auch Signale über die Leitung 68, die die momentane Stellung des Turmes entlang der Ballenreihe 18 angeben. Der Computer 56 gibt auch Angaben entsprechend den Signalen 68 und den Eingaben an der Tastatur 66 an das Videoaufnahmegerät 64 über die Leitung 70 weiter, damit auf dem Videofilm eine einwandfreie Zuordnung zwischen den Aufnahmen und den einzelnen Ballen gegeben ist. Diese Angaben können beispielsweise auf der Tonspur der Videokassette gespeichert werden. Der Computer 56 ist auch in der Lage, die von ihm aufgenommenen Videosignale so zu verarbeiten, daß aus den verarbeiteten Signalen Angaben zu dem Ausmaß der Verunreinigung in der Ballenoberfläche erhalten werden, und diese Angaben können dann ausgedruckt werden, beispielsweise über einen Drucker 72. Die Verarbeitung der Videosignale zu Signalen, welche den Grad der Verunreinigung darstellen, kann auf verschiedenste Art und Weise erfolgen. Beispielsweise kann man davon ausgehen, daß bei einer einheitlichen Baumwollsorte die Oberfläche eine einheitliche Farbe und Streueigenschaft haben wird. Daher müssen die Videosignale bei einem hochwertigen Ballen ohne Verunreinigungen eine im wesentlichen konstante Amplitude aufweisen, welche der Stärke der Beleuchtung proportional ist. Somit können im Computer 56 Schwellenwerte gesetzt werden, beispielsweise über die Tastatur 66, und es werden nur diejenigen Videosignale weiter berücksichtigt, wo die Signalamplitude unterhalb oder oberhalb des angegebenen Schwellwertbereiches liegen. Da die Information eines Videosignals schließlich eine Information für jedes Pixel beinhaltet, und jedes Pixel wiederum eine genaue Flächeneinheit der ausgeleuchteten Ballenoberfläche entspricht. Somit können durch Analyse der Anzahl der Pixel, wo die Signalamplitude außerhalb des Schwellwertbereiches liegt, Angaben zu der Flächenausdehnung der Verunreinigungen in der abgetragenen Schicht gemacht werden, und diese Flächengröße kann als Maß für den Grad der Verunreinigung ausgenutzt werden. Dadurch, daß man vorzugsweise die Oberfläche in drei getrennten Fadenbereichen untersucht, können Fremdfasermaterialien unterschiedlicher Färbungen besser erkannt werden.

Es ist auch möglich, die Videosignale daraufhin zu untersuchen, in welchen zusammenhängenden Pixeln ein vom Durchschnitt abweichendes Bild vorliegt, und nur diejenigen Signale weiterzuverarbeiten, bei denen mehrere zusammenhängende Pixel mit gestörten Signalen feststellbar sind. Hier geht man davon aus, daß die Flächenausdehnung der Verunreinigungen ein gewisses Maß übersteigen muß, um als Verunreinigung anerkannt zu werden. Der Vorteil dieses Verfahrens liegt darin, daß gestörte Bildinformation, die nur einzelne Pixel betreffen, ausgesondert werden kann, da solche Signale eher durch Rauschen oder bestimmte geometrische Unregelmäßigkeiten der Ballenoberfläche verursacht sind.

Schließlich wird in Fig. 1 gezeigt, daß Polarisationsfilter 74 und 76 vor der Videokamera 32 bzw. im Beleuchtungsfeld der Beleuchtungseinrichtung 30 angeordnet sein können, um den Kontrast zwischen Fremdfasern und den erwünschten Baumwollfasern zu erhöhen. Selbstverständlich können diese Polarisationsfilter 74, 76 drehbar oder auswechselbar ausgeführt werden, damit die Oberfläche mit oder ohne polarisiertem Licht und mit verschiedenen Winkeln des polarisierten Lichts untersucht werden kann.

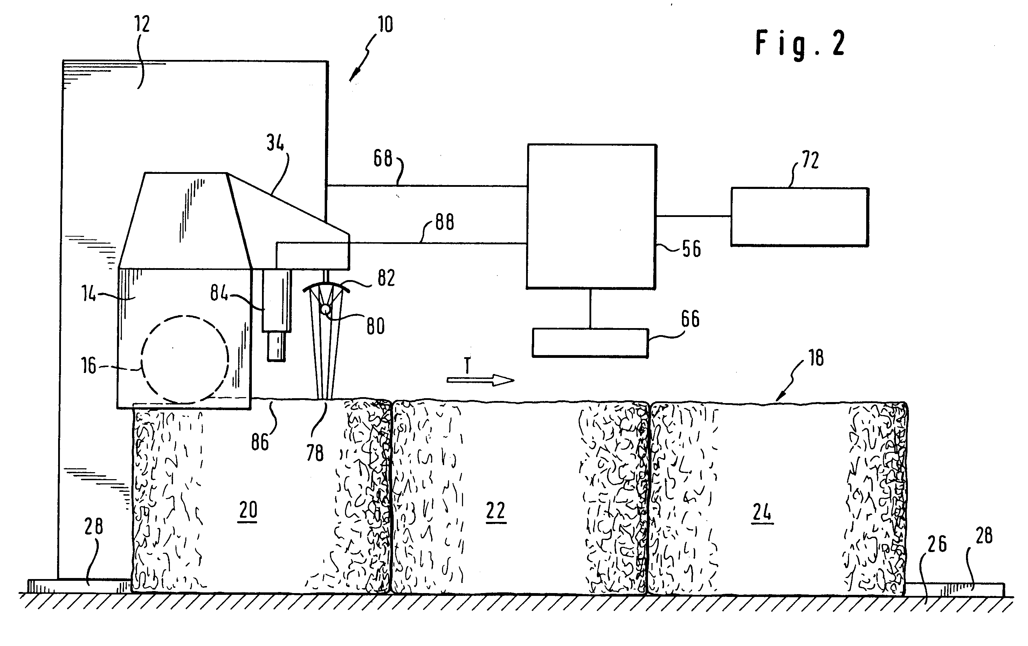

Fig. 2 zeigt eine vergleichbare Anordnung und Teile, die auch in Fig. 1 zu finden sind, sind mit den gleichen Bezugszeichen gekennzeichnet. Der Kürze halber werden hier nur diejenigen Teile extra beschrieben, die von der Ausführung gemäß Fig. 1 abweichen. Die Ausführung nach Fig. 2 arbeitet mit einem Wärmebild der Oberfläche. Zu diesem Zweck wird die Oberfläche in einem Bereich 78 aufgewärmt und zwar mittels eines Infrarotstabes, der sich entlang der Brennlinie eines wärmereflektierenden Hohlspiegels 82 befindet. Auf diese Weise wird ein begrenzter Streifenbereich der Oberfläche aufgewärmt. Durch das Vorbeifahren der Ballenabtragmaschine bewegt sich der aufgewärmte Bereich 78 ständig entlang der Oberfläche der Ballenreihe, wobei vorher aufgewärmte Teile der Oberfläche abkühlen. Die Wärmekamera 84 nimmt dann die Wärmeverteilung entlang eines streifenförmigen Bereiches 86 auf, nachdem dieser Oberflächenbereich eine gewisse Abkühlung erfahren hat. Da die Abkühlung der Fremdfasern und Fremdfolien anders verläuft als die Abkühlung der Baumwollfasern, entstehen im Bereich 86 unterschiedliche Temperaturen, die im Wärmebild deutlich sind. Somit können die Flächenanteile der Bereiche mit abweichender Temperatur ermittelt und als Maß für den Grad der Verunreinigung verwendet werden. Zu diesem Zweck werden die Signale der Wärmekamera 84 über eine Leitung 88 in einen Computer 56 eingelesen und das Ergebnis der Untersuchung wird mit dem Drucker 72 ausgedruckt. Auch hier wird eine Tastatur 66 verwendet, um Information über die einzelnen Ballen einzugeben, und eine Leitung 68 sorgt für die Übertragung von Positionsinformation vom Turm zum Computer 56. Die Wärmekamera 84 kann eine an sich bekannte Wärmekamera sein, es kann aber auch ein neuartiger Kameratyp sein, bei dem die Wärmeverteilung entlang des streifenartigen Bereiches 86 Punkt für Punkt abgetastet wird. Sollte in diesem Beispiel, oder auch in dem Beispiel nach Fig. 1 das Blickfeld der Kamera nicht ausreichen, um die gesamte Breite der Ballenreihe zu erfassen, so können mehrere Kameras nebeneinander angeordnet werden, um die gesamte Breite der Ballenreihe zu erfassen.

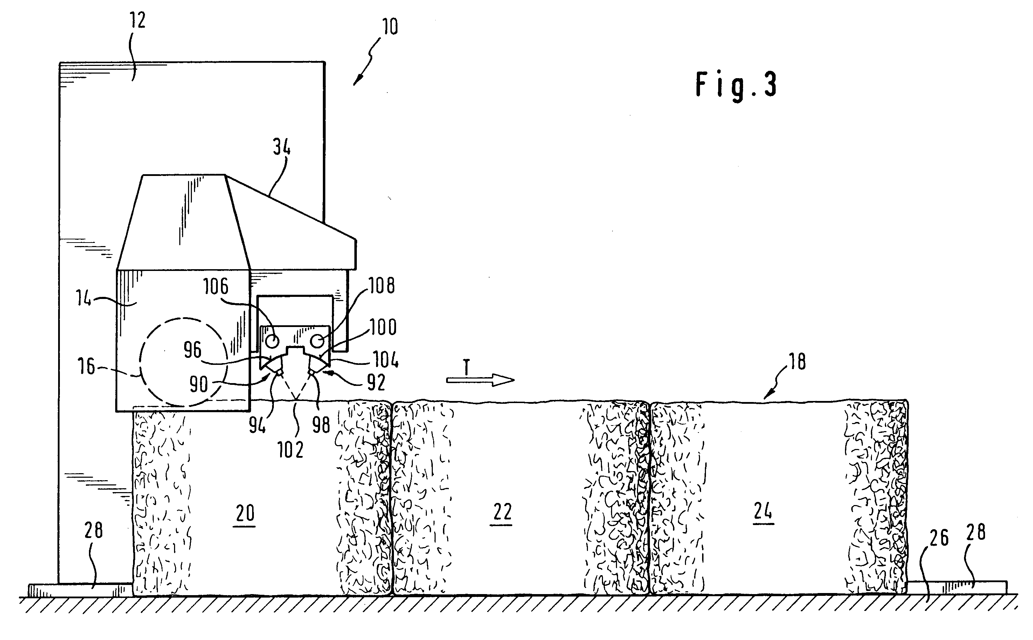

Schließlich zeigt die Fig. 3 eine ähnliche Ballenabtragmaschine zu den Fig. 1 und 2, weshalb auch hier für gleiche Teile die gleichen Bezugszeichen verwendet werden, die Abtastung der Ballenoberfläche findet hier jedoch mittels Ultraschallwellen statt. Zu diesem Zweck ist ein Ultraschallsender 90 und ein Ultraschallempfänger 92 vorgesehen, wobei der Ultraschallsender 90 aus einem piezoelektrischen Schallsender 94 im Brennpunkt eines ein Schallfeld erzeugenden Reflektors 96 besteht. Der Schallempfänger besteht ebenfalls aus einem piezoelektrischen Wandler, der sich im Brennpunkt eines Reflektors 100 befindet. Die vom piezoelektrischen Wandler 94 ausgehenden Strahlwellen werden vom Reflektor 96 in Richtung des Oberflächenbereiches 102 der Ballenreihe gerichtet und dort reflektiert, wobei die reflektierten Strahlungen vom Reflektor 100 auf den piezoelektrischen Wandler 98 konzentriert-werden. Da es schwierig ist, ein Schallfeld über die gesamte Breite der Ballenreihe gleichzeitig zu erzeugen, sind der Sender und der Empfänger 92 auf einem jochartigen Wagen 104 montiert, der entlang zweier Führungsstäbe 106, 108 fahrbar ist, wobei die Führungsstäbe von der Struktur 34 getragen werden.

Die Signale der piezoelektrischen Wandler 94 und 98 werden über nicht gezeigte Leitungen von einem Computer gesteuert bzw. empfangen und dort ausgewertet, um Information über das Vorhandensein von abnormal reflektierenden Bereichen der Oberfläche zu gewinnen.

Die Signalverarbeitung kann so erfolgen wie in der nicht vorveröffentlichten europ. Patentanmeldung EP-A-0364786 angedeutet.

Bei allen Beispielen erfolgt aufgrund der Bewegung des Turmes entlang der Ballenreihe in Pfeilrichtung T eine zeilenweise Abtastung der gesamten freigelegten Oberfläche der Ballenreihe. Wie im Beispiel der Fig. 3 gezeigt, erfolgt auch eine Verschiebung der Abtasteinrichtung entlang der Reihe, man bemüht sich hier daher, die gesamte Oberfläche der Ballenreihe abzutasten. Ain Ende der Ballenreihe wird der Turm 12 entweder in einer Leerbewegung zurückgefahren, um nachfolgend weitere Schichten der Ballenreihe bzw. der einzelnen Ballen abzutragen, oder es wird auf der anderen Seite der Schienen 28 eine weitere Ballenreihe aufgestellt, die auf den Rückweg des Turmes abgearbeitet wird. Zu diesem Zweck ist der Turm 12 in an sich bekannter Weise drehbar ausgeführt.

Eine weitere Möglichkeit besteht darin, anstatt der oben angesprochenen Leerbewegung bei der Rückführbewegung des Turmes, der nicht gedreht wird, ebenfalls Fasermaterial abzutragen. Die Abtasteinrichtung kann bei dieser Rückführbewegung ausgeschaltet werden oder kann auch die Oberfläche der Ballenreihe abtasten. Dies führt zwar dazu, daß die gleiche Oberfläche zweimal abgetastet wird, dies ist jedoch nicht störend, es kann sogar die doppelte Erfassung wirksam ausgenutzt werden, indem die Oberfläche beispielsweise von einer anderen Richtung ausgeleuchtet wird, was zu der Feststellung von bisher "versteckten" Fremdfasern führen kann. Auch kann bei der Abtastung in der einen Richtung die linke Seite der Ballenreihe und bei der Rückführbewegung der rechten Seite die Ballenreihe abgetastet werden (oder umgekehrt). Zu diesem Zweck muß die Abtasteinrichtung lediglich verschiebbar ausgeführt werden, damit sie entlang des Armes zum Abtasten der beiden Seiten der Ballenreihe verschoben werden kann. Eine solche Anordnung hätte eine feinere Auflösung der abgetasteten Fläche zur Folge.

Weiterhin ist es möglich, Abtasteinrichtungen auf beiden Seiten des Armes anzuordnen und entweder gleichzeitig zu betreiben oder nur abwechselnd, je nach Fahrtrichtung.

Bei doppelter Erfassung von Oberflächenbereichen können die statistisch ermittelten Randkörperwerte gemittelt werden.

## Patentansprüche

1. Verfahren zur Ermittlung von mit Fremdkörpern verunreinigtem Fasergut in Faserballen, wobei die in Reihen aufgestellten Faserballen (20,22,24) abgetragen und gleichzeitig nach Fremdkörpern abgetastet werden, dadurch gekennzeichnet, dass
- weiche Fremdkörper in Form von Fremdfasern und Folienresten, insbesondere Verpackungsresten, so wie Jutesackreste, Plastikfolienreste, Schnüre oder Bänder, Lumpen oder Putzfäden ermittelt werden und das Fasergut daraufhin an der Oberfläche der Ballen untersucht wird,
- die Untersuchung durch eine bildliche Aufnahme der Oberfläche wenigstens einiger der durch die Ballenabtragung freigelegten Fasergutschichten über die gesamte Breite der Faserballen erfolgt, und
- das Ausmass der Verunreinigung mit den abgetasteten Fremdkörpern der genannten Form festgehalten und in Zuordnung zu den jeweiligen Ballen (20,22,24) gespeichert wird.

2. Verfahren nach Anspruch 1, dadurch gekennzeichnet, dass das Ausmass der Verunreinigung durch die Anzahl der abgetasteten Fremdkörper und/oder die Ausdehnung der Fremdkörper und/oder die Farbe der Fremdkörper und/oder einen aus der Anzahl, der Ausdehnung und/oder der Farbe abgeleiteten Wert angegeben wird.

3. Verfahren nach einem der vorhergehenden Ansprüche, dadurch gekennzeichnet, daß das Abtasten der freigelegten Oberflächen mittels einer am Turm oder Arm der Ballenabtragmaschine und vorzugsweise in Abtragrichtung vor dem im Arm untergebrachten Abtragorgan angebrachten Abtasteinrichtung durchgeführt wird.

4. Verfahren nach einem der vorhergehenden Ansprüche, dadurch gekennzeichnet, daß die Abtasteinrichtung auf elektrooptischer Basis arbeitet.

5. Verfahren nach Anspruch 4, dadurch gekennzeichnet, daß die Oberfläche der freigelegten Fasergutschichten mit einer Videokamera abgebildet wird, und die so gemessenen Videobilder als Nachweis für die Verunreinigung des Ballens gespeichert werden, beispielsweise in Form eines Videofilms, der auch eine Identifikation des Ballens, z.B. in Form einer Strichkodierung trägt.

6. Verfahren nach Anspruch 5, dadurch gekennzeichnet, daß das nachfolgende Herstellungsverfahren, bei dem das Fasergut zu Garn verarbeitet wird, so durchgeführt wird, daß eine Zuordnung zwischen dem Fasermaterial in den verschiedenen Verarbeitungsstufen und den jeweiligen Faserballen gegeben ist, wodurch bei der Feststellung von erhöhten Fehlern bei der Garnherstellung, die den betreffenden Ballen zugeordneten Videoaufnahmen von einer Betriebsperson gezielt durchgeprüft werden können, um bei mäßigem Aufwand den Nachweis der Verunreinigung sicherzustellen.

7. Verfahren nach Anspruch 4, dadurch gekennzeichnet, daß die Oberfläche der freigelegten Fasergutschichten mit einer Videokamera abgebildet werden, und die so gewonnenen Videosignale elektronisch zu Fremdkörperverunreinigungssignalen verarbeitet und ausgewertet werden.

8. Verfahren nach einem der Ansprüche 4 bis 7, dadurch gekennzeichnet, daß bei jedem Durchgang der Ballenabtragmaschine, d.h. bei dem Abtragen jeder Fasergutschicht von der gesamten Oberfläche der Schicht Videoaufnahmen angefertigt werden.

9. Verfahren nach einem der Ansprüche 4 bis 7, dadurch gekennzeichnet, daß Videoaufnahmen nur während einiger der Durchgänge der Ballenabtragmaschine angefertigt werden.

10. Verfahren nach einem der Ansprüche 4 bis 9, dadurch gekennzeichnet, daß wenigstens der von der Videokamera abgebildete Streifen der Oberfläche bzw. der entsprechende Flächenbereich mit diffusem Licht zumindest im wesentlichen gleichmäßig ausgeleuchtet wird.

11. Verfahren nach Anspruch 10, dadurch gekennzeichnet, daß die Ausleuchtung mittels eines in der Brennlinie eines sich über die Breite des Ballen erstreckenden Hohlspiegels, beispielsweise eines Zylinderspiegels oder Parabolspiegels, angeordneten Leuchtrohres erfolgt, wobei das Leuchtrohr bzw. die Brennlinie des länglichen Hohlspiegels quer zum Ballen und in einer horizontalen Ebene liegt.

12. Verfahren nach Anspruch 11, dadurch gekennzeichnet, daß das Leuchtrohr eine spektrale Strahlungsverteilung aufweist, die sich von wenigstens 400 bis 700 nm erstreckt und vorzugsweise noch weiter in den Infrarotbereich und evtl. auch noch in den UV-Bereich hinein, daß die Videokamera drei Aufnahmeeinrichtungen für unterschiedliche Spektralbereiche aufweist, beispielsweise für die Spektralbereiche blau, grün und rot.

13. Verfahren nach Anspruch 12, dadurch gekennzeichnet, daß die Videosignale der drei Aufnahmeeinrichtungen getrennt ausgewertet werden.

14. Verfahren nach einem der Ansprüche 11, 12 oder 13, dadurch gekennzeichnet, daß eine Einrichtung zur Änderung bzw. Festlegung des Polarisationszustandes des vom Leuchtrohr abgestrahlten und/oder von der Videokamera aufgenommenen Lichtes vorgesehen ist.

15. Verfahren nach einem der Ansprüche 1 bis 3, dadurch gekennzeichnet, daß das Abtasten der freigelegten Oberfläche der Fasergutschichten mittels einer von der Abtragmaschine getragenen Ultraschalleinrichtung vorgenommen wird.

16. Verfahren nach einem der Ansprüche 1 bis 3, dadurch gekennzeichnet, daß die freigelegte Oberfläche des Ballens aufgewärmt wird, beispielsweise mit Infrarotstrahlung oder mit Mikrowellenenergie, und daß ein Wärmebild der Oberfläche angefertigt wird, aus dem der Grad der Verunreinigung ermittelt wird, beispielsweise durch die Flächenteile des Bildes mit gegenüber dem Fasergut erhöhter oder abgesenkter Temperatur.

17. Verfahren nach einem der vorhergehenden Ansprüche, dadurch gekennzeichnet, daß das Ausmaß der Verunreinigung während des Abtragens mit einem vorher bestimmten, vorzugsweise wählbaren Grenzwert verglichen wird, und daß beim Erreichen dieses Grenzwertes entweder für einzelne Ballen oder für eine ganze Ballenreihe eine Anzeige und/oder Alarm ausgelöst wird, damit die Betriebsperson die Gelegenheit hat, das Abtragen zu beenden und auch teilweise abgetragene Ballen auszuwechseln bzw. näher zu untersuchen.

18. Vorrichtung zur Ermittlung von mit Fremdkörpern verunreinigtem Fasergut in Faserballen, welche an einer fahrbaren oder ortsfesten Ballenabtragmaschine befestigt sind, dadurch gekennzeichnet, dass ein Abtastgerät vorgesehen ist, welches die Fremdkörper in Form von Fremdfasern und Folienresten, insbesondere Verpackungsresten, so wie Jutesackreste, Plastikfolienreste, Schnüre oder Bänder, Lumpen oder Putzfäden an der bei der Ballenabtragung schichtweise freigelegten Oberfläche der einzelnen Faserballen über die gesamte Breite der Faserballen detektiert und in Bildern festhält, sowie eine Speichereinrichtung zur Speicherung der durch die Abtastung gewonnenen Bilder, die jeweils einem Ballen zugeordnet sind.

19. Vorrichtung nach Anspruch 18, dadurch gekennzeichnet, daß das Abtastgerät eine Videokamera ist, und daß die Speichereinrichtung ein Videofilm ist.

20. Vorrichtung nach Anspruch 17, dadurch gekennzeichnet, daß eine die freigelegte Oberfläche gleichmäßig ausleuchtende Beleuchtungseinrichtung vorhanden ist.

21. Vorrichtung nach Anspruch 20, dadurch gekennzeichnet, daß die Beleuchtungseinrichtung einen Spektralbereich vom wenigstens etwa 400 nm bis etwa 700 nm umfaßt, der sich vorzugsweise bis in den Infrarot- und ggf. in den UV-Bereich erstreckt, und die Videokamera drei Aufnahmeeinrichtungen umfaßt, welche für die Spektralbereiche blau, grün und rot getrennte Videosignale liefern.

22. Vorrichtung nach Anspruch 18, dadurch gekennzeichnet, daß das Abtastgerät ein kombinierter Ultraschall-Sender/-Emfänger ist.

23. Vorrichtung nach Anspruch 18, dadurch gekennzeichnet, daß eine Wärmequelle zur Aufwartung der Ballenoberfläche vorgesehen ist, und daß das Abtastgerät zur Anfertigung eines Wärmebildes der freigelegten Oberfläche ausgelegt ist.

## Claims

1. A method for determining fibre material contaminated with impurities in fibre bales, whereby the fibre bales (20, 22, 24) placed in rows are taken off and simultaneously scanned for impurities, characterized in that
- soft impurities in form of foreign fibres and residual foils, in particular residual packaging, as well as residual jute bagging, residual plastic foils, ropes or ribbons, rags or cloth threads are determined and the fibre material is thereafter examined on the surface of the bales;
- the examination is carried out by a visual recording of the surface of at least some of the fibre material layers over the whole width of the fibre bales revealed by the bale take-off process, and
- the extent of the impurification with the scanned impurities of the said form is recorded and stored in allocation to the respective bales (20, 22, 24).

2. A method as claimed in claim 1, characterized in that the extent of the impurification is indicated by the number of scanned impurities and/or the extent of the impurities and/or the colour of the impurities and/or a value derived from their number, their extent and/or their colour.

3. A method as claimed in one of the previous claims, characterized in that the scanning of the excavated surfaces is carried out by means of a scanning device provided on the tower or arm of the bale take-off machine and preferably provided in front of take-off member arranged in the arm.

4. A method as claimed in one of the previous claims, characterized in that the scanning device operates on an electrooptical basis.

5. A method as claimed in claim 4, characterized in that the surface of the excavated fibre material layers is recorded with a video camera and that the video images thus recorded are stored as evidence of the impurification of the bale, e.g., in form of a video film, which also carries an identification of the bale, e.g., in form of a bar code.

6. A method as claimed in claim 5, characterized in that the subsequent manufacturing process, in which the fibre material is processed into a yarn, is carried out in such a way that an allocation between the fibre material in the respective processing stages and the respective fibre bale is given, whereupon on encountering an increased rate of errors in the yarn production the video recordings allocated to the respective bale can be checked by an operator in a meaningful manner so as to secure evidence of the impurification by employing moderate means.

7. A method as claimed in claim 4, characterized in that the surface of the excavated fibre material layers are recorded with a video camera and that the video signals thus obtained are processed electronically into impurity signals and then evaluated.

8. A method as claimed in one of the claims 4 to 7, characterized in that in each passage of the bale take-off machine, i.e., during the take-off of each fibre material layer, video recordings are made of the whole surface of each fibre material layer.

9. A method as claimed in one of the claims 4 to 7, characterized in that the video recordings are only made during some of the passages of the bale take-off machine.

10. A method as claimed in one of the claims 4 to 9, characterized in that at least the strip of the surface recorded by the video camera or the respective surface area is lighted by diffuse light at least substantially evenly.

11. A method as claimed in claim 10, characterized in that the lighting is made by a fluorescent lamp provided in the focus line of a concave mirror, e.g., a cylinder mirror or a paraboloidal-type mirror, extending over the width of the bale, with the fluorescent lamp or the focus line of the oblong concave mirror extending transversally to the bale and in a horizontal plane.

12. A method as claimed in claim 11, characterized in that the fluorescent lamp is provided with a spectral radiation distribution which extends from at least 400 to 700 nm and extends preferably even further into the infrared region and, possibly, even into the ultraviolet region, and that the video camera is provided with three recording devices for different spectral regions, e.g., for the spectral ranges blue, green and red.

13. A method as claimed in claim 12, characterized in that the video signals of the three recording devices are evaluated separately.

14. A method as claimed in one of the claims 11, 12 or 13, characterized in that a device is provided for changing or determining the polarization condition of the light emitted by the fluorescent lamp and/or received by the video camera.

15. A method as claimed in one of the claims 1 to 3, characterized in that the scanning of the excavated surface of the fibre material layers is carried out by means of an ultrasonic device carried by the take-off machine.

16. A method as claimed in one of the claims 1 to 3, characterized in that the excavated surface of the bale is heated, e.g., by infrared radiation or with microwave energy, and that a heat image of the surface is made, from which the degree of impurification is determined, e.g. by surface parts of the image with a temperature which is increased or decreased with respect to the fibre material.

17. A method as claimed in one of the previous claims, characterized in that the extent of the impurification is compared during the taking off with a previously defined, preferably selectable threshold value, and that on reaching said threshold value a display and/or an alarm is initiated for individual bales or for a whole row of bales, so that the operator may be given the opportunity to end the taking off and to exchange bales which have been partially taken off or to examine these in closer detail.

18. An apparatus for determining fibre material contaminated with impurities in fibre bales which are attached to a movable or stationary bale take-off machine, characterized in that a scanning device is provided which detects over the whole width of the fibre bales the impurities in form of foreign fibres and residual foils, in particular residual packaging, as well as residual jute bagging, residual plastic foils, ropes or ribbons, rags or cloth threads on the surface of the individual fibre bales which is excavated in layers during the bale take-off and records these in images, as well as a storage device for storing the images gained by the scanning, which images are allocated to a respective bale.

19. An apparatus as claimed in claim 18, characterized in that the scanning device is a video camera and that the storage device is a video film.

20. An apparatus as claimed in claim 17, characterized in that a lighting device is provided which evenly illuminates the excavated surface.

21. An apparatus as claimed in claim 20, characterized in that the lighting device comprises a spectral region of at least 400 nm to approx. 700 nm, which preferably extends up to the infrared region and, optionally, the ultraviolet region, and that the video camera comprises three recording devices which supply separate video signals for the spectral regions blue, green and red.

22. An apparatus as claimed in claim 18, characterized in that the scanning device is a combined ultrasonic transmitter and receiver.

23. An apparatus as claimed in claim 18, characterized in that a heat source is provided for evaluating the bale surface and that the scanning device is arranged for producing a heat image of the excavated surface.

## Revendications

1. Procédé servant à la détection de matière fibreuse contaminée par des corps étrangers dans des balles de fibres, dans lequel les balles de fibres (20, 22, 24), disposées en rangées, sont décortiquées et sont tâtées simultanément en ce qui concerne les corps étrangers,
caractérisé par le fait que
- les corps étrangers tendres, sous forme de fibres étrangères et restes de feuilles, particulièrement des restes d'emballage, ainsi que des restes de sacs de jute, des restes de feuilles de plastique, des ficelles ou des rubans, des chiffons ou étoupes de nettoyage, sont détectés, et la matière fibreuse est ensuite examinée à la surface des balles,
- l'examination est réalisée par une reproduction photographique d'au moins quelques couches de matière fibreuse qui viennent d'être dégagées par l'extraction des balles, sur toute la largeur des balles de fibres, et
- l'importance de la contamination par les corps étrangers tâtés, de ladite forme, est déterminée et est mémorisée en affectation avec chacune des balles (20, 22, 24).

2. Procédé selon revendication 1,
caractérisé par le fait que
l'importance de la contamination est indiquée par le nombre des corps étrangers tâtés et/ou l'expansion des corps étrangers et/ou la couleur des corps étrangers, et/ou par une valeur dérivée du nombre, de l'expansion et/ou de la couleur.

3. Procédé selon l'une des revendications précédentes,
caractérisé par le fait que
le tâtage des surfaces dégagées est effectué à l'aide d'un arrangement tâteur disposé dans la tour ou dans le bras de la machine à décortiquer les balles, et de préférence devant l'organe de décorticage disposé dans le bras, vu dans le sens de décorticage.

4. Procédé selon l'une des revendications précédentes,
caractérisé par le fait que
l'arrangement tâteur travaille sur des bases électro-optiques.

5. Procédé selon revendication 4,
caractérisé par le fait que
la surface des couches de matière fibreuse dégagées est reproduite par une caméra vidéo, et que les images vidéo ainsi mesurées sont enregistrées comme preuves de la contamination de la balle, par exemple sous forme d'un film vidéo qui porte également une identification de la balle, par exemple sous forme d'une codification à bâtonnets.

6. Procédé selon revendication 5,
caractérisé par le fait que
le procédé de fabrication faisant suite, dans lequel la matière fibreuse est traitée pour en faire du fil, est réalisé de telle manière qu'une affectation est donnée entre la matière fibreuse des différentes étapes de traitement et chaque balle de fibres, par quoi, lors de la détection d'un nombre de fautes plus important dans la fabrication du fil, les reproductions vidéo attribuées aux balles concernées peuvent être examinées d'une manière ciblée par une personne d'exécution, afin de déterminer avec sûreté les preuves de contamination avec des frais modérés.

7. Procédé selon revendication 4,
caractérisé par le fait que
la surface des couches de matière fibreuse dégagées est reproduite par une caméra vidéo, et les signaux vidéo ainsi obtenus sont traités électroniquement en signaux de contamination de corps étrangers et sont évalués.

8. Procédé selon l'une des revendications 4 à 7,
caractérisé par le fait que,
à chaque passage de la machine à décortiquer les balles, c'est-à-dire lors du décorticage de chaque couche de matière fibreuse, des reproductions vidéo de la surface totale de la couche sont effectuées.

9. Procédé selon l'une des revendications 4 à 7,
caractérisé par le fait que
les reproductions vidéo ne sont effectuées que pendant quelques passages de la machine à décortiquer les balles.

10. Procédé selon l'une des revendications 4 à 9,
caractérisé par le fait
qu'au moins la bande de surface respectivement la zone de surface correspondante, reproduite par la caméra vidéo, est illuminée au moins essentiellement d'une manière régulière par une lumière diffuse.

11. Procédé selon revendication 10,
caractérisé par le fait que
l'illumination est réalisée à l'aide d'un tube luminescent, disposé dans la ligne focale d'un miroir creux s'étendant sur la largeur de la balle, par exemple un miroir cylindrique ou un miroir parabolique, et où le tube luminescent respectivement la ligne focale du miroir creux oblong est situé perpendiculairement par rapport à la balle et dans un plan horizontal.

12. Procédé selon revendication 11,
caractérisé par le fait que
le tube luminescent possède une répartition spectrale de rayonnement qui s'étend au moins de 400 à 700 nm, et de préférence encore plus loin dans la zone des infrarouges, et éventuellement aussi dans la zone des ultraviolets, que la caméra vidéo possède trois arrangements de reproduction pour des zones spectrales différentes, par exemple pour les zones spectrales bleue, verte et rouge.

13. Procédé selon revendication 12,
caractérisé par le fait que
les signaux vidéo des trois arrangements de reproduction sont évalués séparément.

14. Procédé selon l'une des revendications 11, 12 ou 13,
caractérisé par le fait
qu'un arrangement est prévu pour changer respectivement pour fixer l'état de polarisation de la lumière émise par le tube luminescent et/ou réceptionnée par la caméra vidéo.

15. Procédé selon l'une des revendications 1 à 3,
caractérisé par le fait que
le tâtage de la surface dégagée des couches de matière fibreuse est réalisé à l'aide d'un arrangement à ultrasons porté par la machine à décortiquer.

16. Procédé selon l'une des revendications 1 à 3,
caractérisé par le fait que
la surface dégagée de la balle est réchauffée, par exemple par rayonnement infrarouge ou par énergie micro-ondes, et qu'une image thermique de la surface est produite à l'aide de laquelle le degré de contamination est déterminé, par exemple par les parties de surface de l'image, avec une température augmentée ou abaissée par rapport à la matière fibreuse.

17. Procédé selon l'une des revendications précédentes,
caractérisé par le fait que
l'importance de la contamination est comparée, pendant le décorticage, avec une valeur limite déterminée à l'avance, de préférence pouvant être choisie, et que, lorsque cette valeur limite est atteinte, soit pour des balles particulières ou pour une rangée complète de balles, une indication et/ou une alarme est déclenchée, afin que la personne d'exécution ait l'occasion de terminer le décorticage, et aussi d'échanger des balles partiellement décortiquées respectivement de les examiner de plus près.

18. Procédé servant à la détection de matière fibreuse contaminée par des corps étrangers dans des balles de fibres qui sont fixées sur une machine mobile ou fixe, servant à décortiquer les balles,
caractérisé par le fait
qu'un appareil de tâtage est prévu pour détecter sur toute la largeur des balles de fibres les corps étrangers, sous forme de fibres étrangères et restes de feuilles, particulièrement des restes d'emballage, ainsi que des restes de sacs de jute, des restes de feuilles de plastique, des ficelles ou des rubans, des chiffons ou étoupes de nettoyage, qui se trouvent sur la surface de chaque balle de fibres qui est dégagée couche par couche lors du décorticage des balles, et les maintient en images, et qu'un arrangement de mémoire est prévu pour mémoriser les images obtenues par le tâtage, en affectation avec chacune des balles.

19. Dispositif selon revendication 18,
caractérisé par le fait que
l'appareil de tâtage est une caméra vidéo et que l'arrangement de mémoire est un film vidéo.

20. Dispositif selon revendication 17,
caractérisé par le fait
qu'il y a un arrangement d'illumination qui illumine régulierement la surface dégagée.

21. Dispositif selon revendication 20,
caractérisé par le fait que
l'arrangement d'illumination embrasse une zone spectrale d'au moins 400 nm environ à 700 nm environ, qui s'étend de préférence jusque dans la zone des infrarouges et, le cas échéant, dans la zone des ultraviolets, et la caméra vidéo comprend trois arrangements de reproduction qui livrent des signaux vidéo séparés pour les zones spectrales bleue, verte et rouge.

22. Dispositif selon revendication 18,
caractérisé par le fait que
l'appareil de tâtage est une combinaison émetteur-récepteur à ultrasons.

23. Dispositif selon revendication 18,
caractérisé par le fait
qu'une source de chaleur est prévue pour réchauffer la surface de balle et que l'appareil de tâtage est étudié pour la production d'une image thermique de la surface dégagée.
